# EUROPEAN PATENT APPLICATION

(11) **EP 4 847 553 A1**
(43) Date of publication of application: **02.12.2026**
(21) Application number: 25775851.6
(22) Date of filing: 19.03.2025
(51) Int. Cl.: C08J 3/07, A61F 13/15, A61F 13/53, C08F 8/00, C08J 3/24

(54) **METHOD FOR PRODUCING SOLID POLYMER AND METHOD FOR PRODUCING WATER-ABSORBENT RESIN PARTICLES**

(30) Priority: 28.03.2024 JP 2024055014
(71) Applicant: Sumitomo Seika Chemicals Co., Ltd., Kako-gun, Hyogo 675-0145 (JP)
(72) Inventor: HAYASHI, Yoshiki, Himeji-shi, Hyogo 672-8076 (JP)
(74) Representative: Hasegawa, Kan
(86) International application number: PCT/JP2025/010690
(87) International publication number: WO 2025/205319

(57) **Abstract**

A production method for a solid polymer includes a step of preparing a polymer solution containing a polymer in which a crosslinked structure of a water-absorbent resin has been cleaved, and a step of bringing a poor solvent for the polymer into contact with the polymer solution to precipitate the polymer, thereby obtaining a polymer dispersion liquid in which the solid polymer is dispersed.

## Description

### Technical Field

The present invention relates to a method for producing a solid polymer and a method for producing water-absorbent resin particles.

### Background Art

Generally, disposable sanitary products are configured to contain pulp fibers and a water-absorbent resin between a water-impermeable cover sheet and a water-permeable non-woven fabric, and the water-absorbent resin swells by containing moisture so that excrement can be absorbed. After such sanitary products are used, the sanitary products are treated by incineration or landfilling, but in recent years, the collection and reuse of components from used sanitary products have been examined. For example, Patent Literature 1 discloses a technique for decomposing a water-absorbent resin with a focus on cleavage of a crosslinked portion in the water-absorbent resin, and a technique for collecting a water-soluble recycled polymer obtained by decomposing a water-absorbent resin as an aggregate by crosslinking with a multivalent metal ion.

### Citation List

### Patent Literature

[Patent Literature 1] Japanese Unexamined Patent Publication No. 2020-49398

### Summary of Invention

### Technical Problem

There are cases where a polymer solution is obtained from a collected water-absorbent resin, impurities are then removed from the polymer solution by ultrafiltration to obtain a high-purity polymer solution, and a high-purity solid polymer is subsequently obtained. However, when the amount of the polymer solution is large or the viscosity of the polymer solution is high, the time required for ultrafiltration becomes long, and as a result, a very long time is required to obtain a high-purity solid polymer from the polymer solution.

Accordingly, one aspect of the present invention relates to a method for producing a solid polymer, by which a high-purity solid polymer can be obtained from a polymer solution in a short time.

### Solution to Problem

One aspect of the present invention includes, for example, the following [1] to [6].
[1] A production method for a solid polymer, the production method including:
   a step of preparing a polymer solution containing a polymer in which a crosslinked structure of a water-absorbent resin has been cleaved; and
   a step of bringing a poor solvent for the polymer into contact with the polymer solution to precipitate the polymer, thereby obtaining a polymer dispersion liquid in which the solid polymer is dispersed.
[2] The production method according to [1], wherein the degree of neutralization of the polymer is 80 mol% or more.
[3] The production method according to [1] or [2], wherein the viscosity of the polymer solution at 25°C is 10 mPa·s to 200000 mPa·s.
[4] The production method according to any one of [1] to [3], wherein the content of the poor solvent in the polymer dispersion liquid is 60% by mass or less.
[5] The production method according to any one of [1] to [4], wherein the poor solvent includes at least one selected from the group consisting of alcohols having 1 to 10 carbon atoms, phenols, and ketones.
[6] A production method for water-absorbent resin particles, the production method including a step of crosslinking the solid polymer obtained by the production method according to any one of [1] to [5] to obtain a water-absorbent resin.

### Advantageous Effects of Invention

According to one aspect of the present invention, it is possible to provide a method for producing a solid polymer, by which a high-purity solid polymer can be obtained from a polymer solution in a short time.

### Brief Description of Drawings

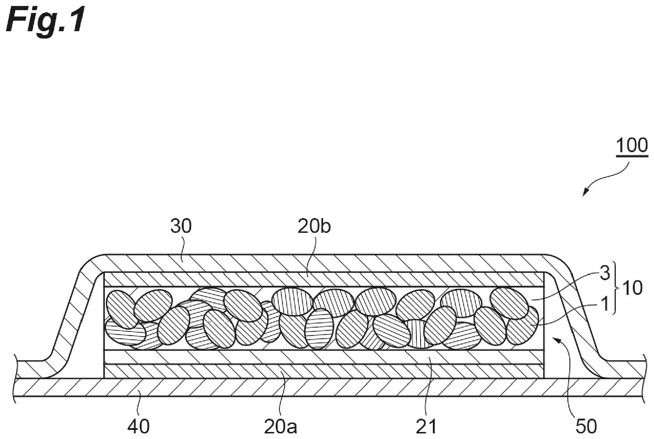

[Fig. 1] A cross-sectional view showing an example of an absorbent article having an absorbent body.

### Description of Embodiments

The present invention is not limited to the following examples.

In the present specification, "(meth)acryl" means both acrylic and methacrylic. The terms "acrylate" and "methacrylate" are also similarly denoted as "(meth)acrylate". The same also applies to other similar terms. The term "(poly)" includes both cases with and without the "poly" prefix. In the numerical value ranges described in a stepwise manner in the present specification, the upper limit or lower limit of a numerical value range in a certain step can be optionally combined with an upper limit or lower limit of a numerical value range in another step. In the numerical value ranges described in the present specification, an upper limit or lower limit of a numerical value range may be replaced with a value shown in examples. The materials exemplified in the present specification may be used alone or in combination of two or more kinds thereof. "Physiological saline" denotes a 0.9 mass% sodium chloride aqueous solution. "Standard sieve" denotes a test sieve (metal mesh sieve) specified in JIS Z 8801-1:2019. Unless otherwise specified, examples and comparative examples are carried out at 1 atm in a normal-temperature and normal-humidity environment, various parameters disclosed in the present specification are values measured in the same environment, and the temperature of various samples is the normal temperature. "One atmosphere" is 101325 Pa, "normal temperature" is 25°C, and "normal humidity" is 50% RH.

An example of the production method for a solid polymer of the present invention includes a step of preparing a polymer solution containing a polymer in which a crosslinked structure of a water-absorbent resin has been cleaved (preparation step), and a step of bringing a poor solvent for the polymer into contact with the polymer solution to precipitate the polymer, thereby obtaining a polymer dispersion liquid in which the solid polymer is dispersed (dispersion step).

### <Water-Absorbent Resin>

The water-absorbent resin may be a water-absorbent resin for recycling. The water-absorbent resin for recycling may be a used water-absorbent resin (that is, a water-absorbent resin in a gel state due to liquid absorption), may be an unused water-absorbent resin (for example, water-absorbent resin waste generated in a step of producing the water-absorbent resin), or may be a mixture of both.

Examples of absorbent articles containing a water-absorbent resin for recycling include diapers (for example, paper diapers), toilet training pants, incontinence pads, sanitary materials (sanitary napkins, tampons, and the like), sweat pads, pet sheets, portable toilet members, and animal excrement treatment materials. The absorbent articles may be used ones. The water-absorbent resin for recycling collected from used water-absorbent articles may form a gel by absorbing liquid during use.

The water-absorbent resin for recycling contains water and a water-absorbent resin. The water-absorbent resin for recycling may contain fibrous substances such as cellulosic fibers and synthetic fibers.

The water-absorbent resin contains, for example, a polymer (crosslinked polymer) of an ethylenically unsaturated monomer. Examples of the ethylenically unsaturated monomers include (meth)acrylic acid and a salt thereof, 2-(meth)acrylamide-2-methylpropanesulfonic acid and a salt thereof, (meth)acrylamide, N,N-dimethyl(meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. The ethylenically unsaturated monomer may include at least one compound selected from the group consisting of acrylic acid and a salt thereof, methacrylic acid and a salt thereof, acrylamide, methacrylamide, and N,N-dimethylacrylamide.

The crosslinked polymer may contain monomer units other than ethylenically unsaturated monomer units. The proportion of the ethylenically unsaturated monomer units (particularly, (meth)acrylic acid and a salt thereof) in the crosslinked polymer may be 70 mol% to 100 mol% with respect to the total amount of the monomer units. The proportion of (meth)acrylic acid and a salt thereof in the ethylenically unsaturated monomer may be 70 mol% to 100 mol%.

The water-absorbent resin contains, for example, a crosslinked polymer having a poly(meth)acrylic acid structure. Examples of such a crosslinked polymer include a polymer obtained by polymerizing a monomer composition containing (meth)acrylic acid and a crosslinking agent capable of forming a covalent bond by reacting with a carboxyl group of (meth)acrylic acid; a polymer obtained by polymerizing a monomer composition containing (meth)acrylic acid to obtain a polymer and treating a surface of the polymer with a crosslinking agent capable of forming a covalent bond by reacting with a carboxyl group of the polymer; and a polymer obtained by polymerizing a monomer composition containing (meth)acrylic acid and a crosslinking agent capable of forming a covalent bond by reacting with a carboxyl group of (meth)acrylic acid to obtain a polymer and treating a surface of the polymer with a crosslinking agent capable of forming a covalent bond by reacting with a carboxyl group of the polymer.

The shape of the water-absorbent resin for recycling is not particularly limited, and may be an irregular lump shape if it is a used gel, or may be an irregular crushed shape, a scale-like shape, a granular shape, or the like if it is an unused one (generally a dry powder).

### <Preparation Step>

In the preparation step, a polymer solution containing a polymer in which a crosslinked structure of a water-absorbent resin has been cleaved is prepared. In the preparation step, for example, a polymer solution containing a polymer in which a crosslinked structure of a water-absorbent resin has been cleaved is obtained by bringing a water-absorbent resin into contact with a base component. The polymer solution may be a mixture of a polymer (a crosslinked polymer in which the crosslinked structure has been cleaved) and a base component. The polymer solution may or may not contain a water-absorbent resin (a crosslinked polymer in which the crosslinked structure has not been cleaved). In a case where the crosslinked polymer contained in the water-absorbent resin is a crosslinked polymer having a poly(meth)acrylic acid structure, the polymer may be a polymer having a poly(meth)acrylic acid structure, and the polymer solution may be a mixture of a polymer having a poly(meth)acrylic acid structure and a base component.

The base component may be an inorganic base component, and may be, for example, at least one inorganic base component selected from the group consisting of sodium hydroxide, ammonia, potassium hydroxide, and calcium hydroxide, or at least one inorganic base component selected from the group consisting of sodium hydroxide, potassium hydroxide, and calcium hydroxide.

The polymer solution may be brought into contact with an acid component or the like to adjust the degree of neutralization of the polymer. The degree of neutralization of the polymer means the proportion of neutralized acidic groups (acidic groups forming a salt) with respect to the total amount of acidic groups in the polymer (including neutralized acidic groups). For example, in a case where the polymer is a partially neutralized product of polyacrylic acid, the degree of neutralization of the polymer means the proportion of carboxylate groups with respect to the total amount of carboxyl groups and carboxylate groups contained in the partially neutralized product of polyacrylic acid.

Since the affinity of the polymer for the poor solvent tends to decrease as the degree of neutralization of the polymer increases, from the viewpoint of easily suppressing the amount of the poor solvent used to precipitate the solid polymer, the degree of neutralization of the polymer contained in the polymer solution may be 40 mol% or more, 50 mol% or more, or 75 mol% or more, and from the viewpoint of further improving the polymer yield, the degree of neutralization may be 80 mol% or more or 90 mol% or more, and from the viewpoint of suppressing the generation of salts due to adjustment of the degree of neutralization, the degree of neutralization may be 95 mol% or less, 85 mol% or less, 80 mol% or less, or 75 mol% or less. From these viewpoints, the degree of neutralization of the polymer contained in the polymer solution may be 40 mol% to 100 mol%, 50 mol% to 100 mol%, 75 mol% to 100 mol%, 80 mol% to 100 mol%, or 90 mol% to 100 mol%.

The viscosity of the polymer solution at 25°C may be 10 mPa·s or more, 50 mPa·s or more, 100 mPa·s or more, 500 mPa·s or more, 1000 mPa·s or more, 1500 mPa·s or more, 3000 mPa·s or more, 5000 mPa·s or more, or 10000 mPa·s or more. The viscosity of the polymer solution at 25°C may be 200000 mPa·s or less, 150000 mPa·s or less, 100000 mPa·s or less, 80000 mPa·s or less, 50000 mPa·s or less, or 30000 mPa·s or less. The viscosity of the polymer solution at 25°C may be 10 mPa·s to 200000 mPa·s, 10 mPa·s to 150000 mPa·s, 10 mPa·s to 100000 mPa·s, 10 mPa·s to 80000 mPa·s, 10 mPa·s to 50000 mPa·s, 10 mPa·s to 30000 mPa·s, 50 mPa·s to 200000 mPa·s, 50 mPa·s to 150000 mPa·s, 50 mPa·s to 100000 mPa·s, 50 mPa·s to 80000 mPa·s, 50 mPa·s to 50000 mPa·s, or 50 mPa·s to 30000 mPa·s, and from the viewpoint of being able to produce a solid polymer in a short time even from a high-viscosity polymer solution that requires a long time for ultrafiltration, the viscosity may be 500 mPa·s to 200000 mPa·s, 500 mPa·s to 150000 mPa·s, 500 mPa·s to 100000 mPa·s, 500 mPa·s to 80000 mPa·s, 500 mPa·s to 50000 mPa·s, 500 mPa·s to 30000 mPa·s, 1000 mPa·s to 200000 mPa·s, 1000 mPa·s to 150000 mPa·s, 1000 mPa·s to 100000 mPa·s, 1000 mPa·s to 80000 mPa·s, 1000 mPa·s to 50000 mPa·s, 1000 mPa·s to 30000 mPa·s, 10000 mPa·s to 200000 mPa·s, 10000 mPa·s to 150000 mPa·s, 10000 mPa·s to 100000 mPa·s, 10000 mPa·s to 80000 mPa·s, 10000 mPa·s to 50000 mPa·s, or 10000 mPa·s to 30000 mPa·s.

The concentration of the polymer in the polymer solution may be, for example, 0.1% by mass or more, 0.5% by mass or more, or 1% by mass or more, and may be 25% by mass or less, 15% by mass or less, 10% by mass or less, 7% by mass or less, or 5% by mass or less. The concentration of the polymer in the polymer solution may be 0.1% by mass to 25% by mass, 0.1% by mass to 15% by mass, 0.1% by mass to 10% by mass, 0.5% by mass to 7% by mass, or 1% by mass to 5% by mass.

### <Dispersion Step>

In the dispersion step, a poor solvent for the polymer is brought into contact with the polymer solution to precipitate the polymer dissolved in the polymer solution, thereby obtaining a polymer dispersion liquid in which the solid polymer is dispersed. The poor solvent is a solvent in which the solubility of the polymer in 100 g of the solvent at 25°C is less than 0.1 g. The poor solvent may be a water-soluble organic solvent.

Examples of a method of bringing a poor solvent for the polymer into contact with the polymer solution include a method of adding the poor solvent to the polymer solution and a method of adding the polymer solution to the poor solvent. In a case where a poor solvent is added to the polymer solution, the entire planned amount of the poor solvent to be used may be added to the polymer solution at once, or may be added to the polymer solution over time. Similarly, in a case where a polymer-poor solution is added to a poor solvent, the entire polymer solution may be added to the poor solvent at once, or may be added to the poor solvent over time. The expression "added over time" denotes that a poor solvent (or a polymer solution) is continuously or intermittently added to a polymer solution (or a poor solvent) over a predetermined time. From the viewpoint of suppressing the amount of the poor solvent to be used, a method of adding a poor solvent to the polymer solution over time may be employed.

Examples of the poor solvent include alcohols having 1 to 10 carbon atoms, phenols, ketones, ethers, and nitriles. From the viewpoint of easily obtaining a solid polymer with higher purity, the poor solvent may be at least one selected from the group consisting of alcohols having 1 to 10 carbon atoms, phenols, and ketones.

Examples of the alcohols having 1 to 10 carbon atoms include methanol, ethanol, propanol, butanol, pentanol, ethylene glycol, and glycerin. Examples of the phenols include phenol, cresol, dibutylhydroxytoluene, and eugenol. Examples of the ketones include acetone, methyl ethyl ketone, and diethyl ketone. Examples of the ethers include dimethyl ether, ethyl methyl ether, diethyl ether, furan, and tetrahydrofuran. Examples of the nitriles include acrylonitrile. The poor solvent may be at least one selected from the group consisting of methanol, ethanol, and acetone.

In a case where the method of adding a poor solvent to the polymer solution over time is used to bring the polymer solution and the poor solvent into contact with each other, the concentration of the poor solvent in the polymer dispersion liquid to be finally obtained may be 60% by mass or less, 50% by mass or less, 40% by mass or less, 30% by mass or less, or 20% by mass or less from the viewpoint of easily obtaining the solid polymer in a shorter time. The concentration of the poor solvent in the polymer dispersion liquid may be 10% by mass or more or 20% by mass or more. The content of the poor solvent in the polymer dispersion liquid may be 10% by mass to 60% by mass or 20% by mass to 50% by mass.

In addition, in a case where the method of adding a poor solvent to the polymer solution over time is used to bring the polymer solution and the poor solvent into contact with each other, the addition rate of the poor solvent may be 30 mass%/min or less or 25 mass%/min or less with respect to the polymer solution from the viewpoint that the solid polymer is easily precipitated in the form of small particles so that impurities are difficult to remain inside the precipitated solid polymer (that is, the purity of the solid polymer becomes higher), and may be 0.1 mass%/min or more, 1 mass%/min or more, or 5 mass%/min or more from the viewpoint of reducing the time required for dropwise addition. The dropping rate of the poor solvent may be 0.1 mass%/min to 30 mass%/min, 0.1 mass%/min to 25 mass%/min, 1 mass%/min to 30 mass%/min, 1 mass%/min to 25 mass%/min, 5 mass%/min to 30 mass%/min, or 5 mass%/min to 25 mass%/min.

The impurity removal rate in the dispersion step may be 75% by mass or more, 80% by mass or more, 85% by mass or more, or 90% by mass or more. The impurity removal rate can be calculated by the method described in the Examples below.

The polymer yield in the dispersion step may be 75% by mass or more, 80% by mass or more, 85% by mass or more, or 90% by mass or more. The polymer yield can be calculated by the method described in the Examples below.

The shape of the solid polymer dispersed in the polymer dispersion liquid may be a particle shape or a lump shape.

The weight-average molecular weight of the solid polymer in the polymer dispersion liquid may be 1,700,000 or more, 1,800,000 or more, or 1,900,000 or more, and may be 2,500,000 or less or 2,400,000 or less. The weight-average molecular weight of the polymer in the polymer dispersion liquid may be 1,700,000 to 2,500,000 or 1,800,000 to 2,400,000.

### <Crosslinking Step>

After the polymer dispersion liquid is obtained, the solid polymer obtained by removing the supernatant liquid from the polymer dispersion liquid may be crosslinked to obtain a water-absorbent resin. That is, another embodiment of the present invention is a production method for water-absorbent resin particles, the production method including a step of crosslinking the solid polymer in the polymer dispersion liquid obtained by the above-described production method to obtain a water-absorbent resin (crosslinking step).

After the solid polymer is obtained and before the crosslinking step is carried out, dilution, heating, addition of a salt (a salt containing a monovalent cation), or the like may be performed on the polymer dispersion liquid from the viewpoint of reducing the viscosity of the polymer dispersion liquid.

After the solid polymer is obtained and before the solid polymer is crosslinked, a step of adjusting the degree of neutralization to a predetermined level may be carried out. After the solid polymer is obtained and before the solid polymer is crosslinked, a sterilization treatment may be performed on the solid polymer.

In the crosslinking step, for example, a solution containing a crosslinking agent capable of forming a covalent bond with a functional group contained in the solid polymer is prepared, and the solid polymer is crosslinked through a covalent bond to obtain a crosslinked polymer. In the crosslinking step, the polymer dispersion liquid may undergo gelation. In that case, the entire polymer dispersion liquid loses fluidity, and a gel containing a crosslinked polymer and water is formed. In a case where the crosslinking proceeds to the extent that a gel is formed, it is particularly easy to obtain water-absorbent resin particles having high water absorption performance and a small amount of soluble content.

Examples of the functional group contained in the solid polymer include a carboxyl group. In a case where the solid polymer has a carboxyl group, the solid polymer is crosslinked by a reaction between the carboxyl group and a crosslinking agent and/or a reaction between carboxyl groups. The covalent bond may be at least one selected from the group consisting of an ester bond, a thioester bond, an amide bond, an ether bond, and a carbon-carbon bond. The solid polymer may be crosslinked through, for example, at least one group selected from the group consisting of a carboxylic acid ester group, a thioester group, an amide group, an acid anhydride group, an oxyalkylene group, and an oxyarylene group.

Examples of the crosslinking agent include aliphatic polyhydric alcohols such as (poly)ethylene glycol, (poly)propylene glycol, (poly)glycerin, and pentaerythritol; glycidyl compounds such as (poly)ethylene glycol diglycidyl ether, (poly)propylene glycol diglycidyl ether, and (poly)glycerin diglycidyl ether; bisacrylamide compounds such as N,N'-methylene bis(meth)acrylamide; allylated starch; diallyl phthalate; N,N',N''-triallylisocyanurate; divinylbenzene; ethylenediamine, polyethyleneimine, and glycidyl (meth)acrylate.

The amount of the crosslinking agent, per 100 parts by mass of the solid polymer, may be 0.0001 parts by mass or more, 0.001 parts by mass or more, or 0.005 parts by mass or more, and may be 10 parts by mass or less, 10 parts by mass or less, or 5 parts by mass or less. The amount of the crosslinking agent may be 0.0001 parts by mass to 15 parts by mass, 0.001 parts by mass to 10 parts by mass, or 0.005 parts by mass to 5 parts by mass per 100 parts by mass of the solid polymer.

The reaction temperature for crosslinking the solid polymer is appropriately set depending on the kind, the amount, and the like of the crosslinking agent to be used, but may be 50°C or higher, 80°C or higher, or 100°C or higher, and may be 220°C or lower, 200°C or lower, or 180°C or lower. The reaction temperature for crosslinking the solid polymer may be 50°C to 220°C, 80°C to 200°C, or 100°C to 180°C.

The reaction time for crosslinking the solid polymer is appropriately set depending on the kind and amount of the crosslinking agent to be used, the reaction temperature, and the like, but may be 1 minute to 200 minutes or 5 minutes to 150 minutes.

After the crosslinked polymer is obtained through the crosslinking step, a surface crosslinking step of performing surface crosslinking of the crosslinked polymer may be carried out. The surface crosslinking can be performed, for example, by adding a crosslinking agent for performing the surface crosslinking (surface crosslinking agent) to the crosslinked polymer and carrying out the reaction.

The surface crosslinking agent may be a compound having two or more reactive functional groups having reactivity with the crosslinked polymer. The surface crosslinking agent may be the same as or different from the crosslinking agent in the crosslinking step.

The reactive functional group of the surface crosslinking agent may be a carbonate group, an alcoholic hydroxy group, an epoxy group, a halogeno group in a haloepoxy compound, an isocyanate group, an oxetanyl group, an oxazoline group, or a combination thereof. The carbonate group can react with two other molecules, and thus is regarded as two reactive functional groups.

Examples of the surface crosslinking agent containing a carbonate group include alkylene carbonate (ethylene carbonate and the like). Examples of surface crosslinking agents containing an alcoholic hydroxyl group include polyol compounds such as ethylene glycol, propylene glycol, 1,4-butanediol, trimethylolpropane, glycerin, polyoxyethylene glycol, polyoxypropylene glycol, and polyglycerin, and hydroxyalkylamide compounds (bis[N,N-di(β-hydroxyethyl)]adipamide and the like). Examples of the surface crosslinking agent containing two or more epoxy groups include (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. Examples of haloepoxy compounds containing an epoxy group and a halogeno group include epichlorohydrin, epibromohydrin, and α-methylepichlorohydrin. Examples of the surface crosslinking agent containing an isocyanate group include 2,4-tolylene diisocyanate and hexamethylene diisocyanate. Examples of surface crosslinking agents containing an oxetanyl group include 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol.

The amount of the surface crosslinking agent may be 0.001 parts by mass or more, 0.005 parts by mass or more, or 0.01 parts by mass or more, and may be 5 parts by mass or less, 3 parts by mass or less, or 1 part by mass or less per 100 parts by mass of the crosslinked polymer. The amount of the crosslinking agent may be 0.001 parts by mass to 5 parts by mass, 0.005 parts by mass to 3 parts by mass, or 0.01 parts by mass to 1 part by mass per 100 parts by mass of the crosslinked polymer.

A regenerated water-absorbent resin containing a crosslinked polymer is formed by a method including removing water from the crosslinked polymer obtained through any optional surface crosslinking step. In a case where the reaction solution subjected to the crosslinking step is an aqueous solution, the water-absorbent resin particles are formed by a method including a drying step of removing water from a lumpy crosslinked polymer formed by gelation of the reaction solution itself and drying the crosslinked polymer to form a dried product, and a pulverization step of pulverizing the dried product.

A method of drying the crosslinked polymer may be, for example, a typical method such as a pressing method such as centrifugation, dehydration using an organic solvent, natural drying, heat drying, air drying, freeze drying, or a combination thereof. From the viewpoint of efficiently removing water, the heating temperature for drying may be 80°C to 220°C, 90°C to 200°C, or 100°C to 180°C.

The moisture content of the dried product may be, for example, 20% by mass or less, 10% by mass or less, or 5% by mass or less. The moisture content of the dried product means the proportion of moisture in the dried product based on the total amount of the dried product. The moisture content of the dried product can be determined as the difference in mass of the crosslinked polymer before and after heating, when the crosslinked polymer containing water is heated at 200°C for 2 hours.

Before drying the crosslinked polymer, a coarsely crushed product including structures of a somewhat small size may be formed by coarsely crushing the crosslinked polymer. Water can be efficiently removed by forming the coarsely crushed product. The structures constituting the coarsely crushed product can be, for example, elongated structures, granular structures (particles), or a combination thereof. The coarsely crushed product may include a plurality of structures having a shape that enables them to pass through a circular hole having a diameter of 10 mm or 7 mm. The elongated structure may be bent, and in a case where the maximum width thereof is 10 mm or less, it can be said that the elongated structure has a shape that enables the structure to pass through a circular hole having a diameter of 10 mm. The granular structure (particle) may be irregularly shaped and may have a shape that enables the structure to pass through a circular hole having a diameter of 10 mm while changing the orientation. Examples of a coarse crusher for coarsely crushing the crosslinked polymer include a kneader (for example, a pressure type kneader or a double-arm type kneader), a meat chopper, a cutter mill, and a pharma mill.

The dried product is pulverized to form water-absorbent resin particles. The pulverization method is not particularly limited. For example, the dried product can be pulverized using a pulverizer such as a centrifugal pulverizer, a roller mill, a stamp mill, a jet mill, a high-speed rotary pulverizer, or a container drive type mill.

The water-absorbent resin particles obtained by pulverization may be classified. The classification denotes an operation of dividing a particle group (powder) into two or more particle groups having different particle size distributions. Some of the water-absorbent resin particles after the classification may be pulverized and classified again.

The classification method is not particularly limited, but may be, for example, screen classification or airflow classification. The screen classification is a method of classifying particles on a screen into particles that pass through the openings of the screen and particles that do not pass through the openings by vibrating the screen. The screen classification can be performed by using, for example, a vibrating screen, a rotary sifter, a cylindrical stirring screen, a blower sifter, or a Ro-tap shaker. The airflow classification is a method of classifying particles using the flow of air.

The median particle diameter of the powder of the crosslinked polymer obtained by performing pulverization and, as necessary, classification may be, for example, 200 µm to 500 µm or 300 µm to 500 µm. The particle size distribution may be adjusted by mixing two or more powders having different median particle diameters, which are obtained by the classification.

Fig. 1 is a cross-sectional view showing an example of an absorbent article having an absorbent body containing water-absorbent resin particles. An absorbent article 100 shown in Fig. 1 includes a water-absorbent sheet 50 having a film-like absorbent body 10, a liquid permeable sheet 30, and a liquid impermeable sheet 40.

The water-absorbent sheet 50 has an absorbent body 10 containing a powder of water-absorbent resin particles 1 and two core wrap sheets 20a and 20b. The absorbent body 10 is disposed inside the core wrap sheets 20a and 20b. The shape of the absorbent body 10 is retained by being sandwiched between the two core wrap sheets 20a and 20b. The core wrap sheets 20a and 20b may be two sheets, a single folded sheet, or a single bag body. A sheet member having no other constituent members provided on the outside of the core wrap sheets 20a and 20b that wrap the absorbent body 10 is also particularly referred to as a water-absorbent sheet.

The absorbent body 10 is a constituent member which mainly contains a powder of the water-absorbent resin particles 1 and whose shape is retained to be constant. The absorbent body 10 may or may not include a fibrous substance 3 in addition to the powder of the water-absorbent resin particles 1. The content of the water-absorbent resin particles 1 in the absorbent body 10 may be 50% by mass or more and 100% by mass or less, 60% by mass or more and 100% by mass or less, 70% by mass or more and 100% by mass or less, 80% by mass or more and 100% by mass or less, or 90% by mass or more and 100% by mass or less with respect to the mass of the absorbent body 10.

The thickness of the absorbent body 10 may be, for example, 20 mm or less, 15 mm or less, 10 mm or less, 5 mm or less, 4 mm or less, or 3 mm or less, or may be 0.1 mm or more or 0.3 mm or more. The thickness of the absorbent body 10 may be 0.1 mm or more and 20 mm or less. The mass of the absorbent body 10 per unit area may be 1000 g/m² or less, 800 g/m² or less, or 600 g/m² or less, or may be 100 g/m² or more.

The fibrous substance 3 can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous substance may be hydrophilic fibers (for example, pulp).

The absorbent body 10 may further contain an inorganic powder (for example, amorphous silica), a deodorant, an antibacterial agent, a fragrance, or the like. In a case where the water-absorbent resin particles 1 include inorganic particles, the absorbent body 10 may contain an inorganic powder in addition to the inorganic particles in the water-absorbent resin particles 1.

The water-absorbent sheet 50 may further have an adhesive 21 interposed between the core wrap sheet 20a and the absorbent body 10. An adhesive layer may be interposed between the core wrap sheets 20a and 20b on both sides and the absorbent body 10. The adhesive 21 is not particularly limited, and may be, for example, a hot melt adhesive.

The core wrap sheets 20a and 20b may be, for example, non-woven fabrics. The two core wrap sheets 20a and 20b may be the same or different non-woven fabrics. The non-woven fabrics may be non-woven fabrics (short-fiber non-woven fabrics) formed of short fibers (that is, staple fibers), or may be non-woven fabrics (long-fiber non-woven fabrics) formed of long fibers (that is, filaments). Although not limited thereto, the staples generally may have a fiber length of several hundred mm or less.

The core wrap sheets 20a and 20b may be thermal bonded non-woven fabrics, air-through non-woven fabrics, resin bonded non-woven fabrics, spunbond non-woven fabrics, melt-blown non-woven fabrics, air-laid non-woven fabrics, spunlace non-woven fabrics, point-bonded non-woven fabrics, or a laminate containing two or more non-woven fabrics selected from these non-woven fabrics.

The non-woven fabric used as the core wrap sheets 20a and 20b may be a non-woven fabric formed of synthetic fibers, natural fibers, or a combination thereof. Examples of the synthetic fibers include fibers containing synthetic resins selected from polyolefin such as polyethylene (PE) and polypropylene (PP), polyester such as polyethylene terephthalate (PET), polytrimethylene terephthalate (PTT), and polyethylene naphthalate (PEN), polyamide such as nylon, and rayon. Examples of the natural fibers include fibers containing cotton, silk, hemp, or pulp (cellulose). The fibers forming the non-woven fabrics may be polyolefin fibers, polyester fibers, or a combination thereof. The core wrap sheets 20a and 20b may be tissue paper.

The water-absorbent sheet 50 may be used for producing various other absorbent articles. Examples of the absorbent articles include diapers (for example, paper diapers), toilet training pants, incontinence pads, sanitary materials (sanitary napkins, tampons, and the like), sweat pads, pet sheets, portable toilet members, and animal excrement treatment materials. The absorbent bodies constituting these absorbent articles frequently move or deform due to the movement or the like of a user of the absorbent article.

The liquid permeable sheet 30 is disposed at the position of the outermost layer on a side where the liquid to be absorbed enters. The liquid permeable sheet 30 is disposed outside the core wrap sheet 20b in a state of being in contact with the core wrap sheet 20b. The liquid impermeable sheet 40 is disposed at the position of the outermost layer on the side opposite to the liquid permeable sheet 30 in the absorbent article 100. The liquid impermeable sheet 40 is disposed outside the core wrap sheet 20a in a state of being in contact with the core wrap sheet 20a. The liquid permeable sheet 30 and the liquid impermeable sheet 40 have main surfaces wider than the main surface of the water-absorbent sheet 50, and outer edges of the liquid permeable sheet 30 and the liquid impermeable sheet 40 extend around the absorbent body 10 and the core wrap sheets 20a and 20b. However, the size relationship between the absorbent body 10, the core wrap sheets 20a and 20b, the liquid permeable sheet 30, and the liquid impermeable sheet 40 is not particularly limited, and is appropriately adjusted depending on the applications of the absorbent article or the like.

The liquid permeable sheet 30 may be a non-woven fabric. The non-woven fabric used as the liquid permeable sheet 30 may have appropriate hydrophilicity from the viewpoint of the liquid absorption performance of the absorbent article. From this viewpoint, the liquid permeable sheet 30 may be a non-woven fabric having a hydrophilicity of 5 to 200, which is measured in accordance with the measuring method of the Japan Technical Association of Pulp and Paper Industry Test Method No. 68 (2000). The hydrophilicity of the non-woven fabric may be 10 to 150. For the details of Pulp and Paper Test Method No. 68, reference can be made to, for example, WO2011/086843.

The non-woven fabric having hydrophilicity may be formed of fibers such as rayon fibers exhibiting an appropriate hydrophilicity, or may be formed of fibers obtained by performing a hydrophilic treatment on hydrophobic chemical fibers such as polyolefin fibers and polyester fibers. Examples of a method of obtaining a non-woven fabric including hydrophobic chemical fibers that have been subjected to a hydrophilic treatment include a method of obtaining a non-woven fabric by a spunbond method using a mixture obtained by mixing a hydrophilic agent with hydrophobic chemical fibers, a method of incorporating a hydrophilic agent in preparing a spunbond non-woven fabric with hydrophobic chemical fibers, and a method of impregnating a spunbond non-woven fabric obtained using hydrophobic chemical fibers with a hydrophilic agent. As the hydrophilic agent, an anionic surfactant such as an aliphatic sulfonate or a higher alcohol sulfate ester salt, a cationic surfactant such as a quaternary ammonium salt, a nonionic surfactant such as a polyethylene glycol fatty acid ester, a polyglycerin fatty acid ester, or a sorbitan fatty acid ester, a silicone surfactant such as polyoxyalkylene-modified silicone, and a stain release agent formed of a polyester-based, polyamide-based, acrylic-based, or urethane-based resin, or the like are used.

From the viewpoint of imparting satisfactory liquid permeability, flexibility, strength, and cushioning properties to the absorbent article and from the viewpoint of accelerating the liquid permeation rate of the absorbent article, the basis weight (mass per unit area) of the non-woven fabric used as the liquid permeable sheet 30 may be 5 g/m² to 200 g/m², 8 g/m² to 150 g/m², or 10 g/m² to 100 g/m². The thickness of the liquid permeable sheet 30 may be 20 µm to 1400 µm, 50 µm to 1200 µm, or 80 µm to 1000 µm.

The liquid impermeable sheet 40 prevents a liquid absorbed by the absorbent body 10 from leaking to the outside from the liquid impermeable sheet 40 side. The liquid impermeable sheet 40 may be a resin sheet or a non-woven fabric. The resin sheet may be a sheet formed of a synthetic resin such as polyethylene, polypropylene, and polyvinyl chloride. The non-woven fabric may be a spunbond/melt-blown/spunbond (SMS) non-woven fabric in which a waterproof melt-blown non-woven fabric is sandwiched between high-strength spunbond non-woven fabrics. The liquid impermeable sheet 40 may be a composite sheet of a resin sheet and a non-woven fabric (for example, a spunbond non-woven fabric, a spunlace non-woven fabric). The liquid impermeable sheet 40 may have breathability from the viewpoint that dampness generated at the time of wearing is reduced, and thereby discomfort to a wearer can be reduced. As the liquid impermeable sheet 40 having breathability, it is possible to use, for example, a sheet of low-density polyethylene (LDPE) resin.

From the viewpoint of ensuring the flexibility so that the feeling of wearing the absorbent article is not impaired, the basis weight (mass per unit area) of the liquid impermeable sheet 40 may be 10 g/m² to 50 g/m².

### Examples

Hereinafter, the present invention will be described in more detail with reference to the Examples.

### <Evaluation Method>

The viscosity, weight-average molecular weight, solid content fraction, impurity removal rate, polymer yield, and time required were measured according to the following evaluation methods. The measurement results are as shown in Table 1.

### [Viscosity]

The viscosity of the polymer solution was measured using a rheometer (manufactured by TA Instruments, model number: AR 2000ex) under the following conditions. The viscosity is a steady-flow viscosity at a shear rate of 4.6 s⁻¹.
· Jig: 2° 40 mm cone plate
· Measurement temperature: 25°C

### [Weight-Average Molecular Weight Mw]

The weight-average molecular weight of the solid polymer was measured by light scattering GPC under the following conditions.
· Autosampler: Autosampler AS-11 (manufactured by FLOM Co., Ltd.)
· Degassing device: Gastorr AG-16 (manufactured by FLOM Co., Ltd.)
· Liquid delivery unit: LC-10AD (manufactured by Shimadzu Corporation)
· Column: OHpak SB-807HQ, SB-806HQ, SB-804HQ (Shodex series, manufactured by Resonac Corporation)
· Detector: Triple Detector TDA 302 (manufactured by Viscotec)
· Carrier solvent: NaNO₃ (0.2 mol/L)/methylparaben (2 mmol/L)/distilled water
· Measurement conditions: injection volume 500 µL, flow rate 0.5 mL/min, column/detector temperature 40°C, dn/dC 0.2270
· 0.8 µm membrane filter (manufactured by Advantec)

### <Sample Preparation Procedure and Measurement Procedure>

A solid polymer containing 0.01 g of solid content was placed in a 300 mL beaker, and a 0.2 M NaNO₃ aqueous solution as an eluent was added thereto so that the total amount reached 100 g, and the solution was stirred at 250 rpm for 1 hour. In a case where the pH of the sample solution was not neutral, the pH was adjusted to 7 using hydrochloric acid (manufactured by Nacalai Tesque, Inc., 1 mol/L hydrochloric acid) at this time. The sample solution after stirring was measured by GPC using a filtrate obtained by passing it through a filter syringe having a pore size of 0.8 µm.

### [Solid Content Fraction]

An aluminum pan was placed in a simultaneous thermogravimetric and differential thermal analyzer (manufactured by Hitachi High-Tech Corporation, TG/DTA7220), and the weight of the pan at normal temperature was measured to perform zero-point correction. Thereafter, an arbitrary amount (approximately 5 mg to 40 mg) of a sample was placed on the pan, and the weight (C [g]) was measured. The pan was heated at 5°C/min under a normal-temperature measurement atmosphere purged with nitrogen at 200 mL/min. Subsequently, the weight of the sample (D [g]) at the point when the pan reached 200°C was measured, and the solid content fraction was calculated by the following equation. Solid content fraction (% by mass) = (D/C) × 100

### [Impurity Removal Rate]

The polymer solution before purification (before polymer precipitation) was diluted with ion-exchanged water until the viscosity became low enough to allow ultrafiltration. The polymer concentration in the diluted polymer solution was defined as a₁ (% by mass), the concentration of substances other than the polymer (impurity concentration) was defined as b₁ (% by mass), and the solid content fraction (a₁ + b₁ (% by mass)) of the diluted polymer solution (sample) was measured using TG/DTA7220. Next, the diluted polymer solution was placed in a centrifugal concentrator with a molecular weight cut-off of 3000, and centrifuged at 2000 rpm for 30 min. The obtained filtrate was used as a sample, and its solid content fraction ({b₁/(100 - a₁)} × 100 (% by mass)) was measured using TG-DTA. Since simultaneous equations for a₁ and b₁ were obtained from the measurement results, a₁ and b₁ were calculated, and the impurity ratio b₁/a₁ of the polymer before purification was calculated. The same operation was performed on the polymer dispersion liquid after purification (in the comparative example, the polymer solution after ultrafiltration), and the polymer concentration a₂ (% by mass) and the concentration of substances other than the polymer b₂ (% by mass) contained in the diluted polymer dispersion liquid or the diluted polymer solution after ultrafiltration were calculated, and the impurity ratio b₂/a₂ of the polymer after purification was calculated. Subsequently, the impurity removal rate was calculated by the following equation. $Impurity removal rate \left(% by mass\right) = 100 - \left\{\left({b}_{2}/{a}_{2}\right)/\left({b}_{1}/{a}_{1}\right)\right\} \times 100$

### [Polymer Yield]

The weight after draining the polymer dispersion liquid (in the comparative example, the polymer solution after ultrafiltration) by allowing it to stand on a standard sieve with an opening of 75 µm for 30 minutes was defined as the mass c (g) of the precipitate (in the comparative example, the polymer). Subsequently, a portion of the drained precipitate was sampled, and a 30-fold dilution prepared using ion-exchanged water in an amount 29 times the weight of the obtained sample was used as a sample, and its solid content fraction was measured using TG/DTA7220. The solid content fraction of the precipitate d (% by mass) was calculated by multiplying the obtained solid content fraction by 30, and the polymer yield was calculated by the following equation. $Polymer yield \left(mol%\right) = 100 \times \left(B/A\right)$

Here, in the above equation, A is the total amount (mol) of monomer units constituting the water-absorbent resin particles subjected to cleavage of the crosslinked structure. In the examples and comparative examples of the present application, water-absorbent resin particles manufactured by Sumitomo Seika Chemicals Co., Ltd. (Aqua Keep, sodium polyacrylate with a degree of neutralization of 75 mol%) are used as described below, and therefore A (mol) corresponds to the total amount (mol) of monomer units constituting the sodium polyacrylate with a degree of neutralization of 75 mol%. Note that the monomer units constituting sodium polyacrylate with a degree of neutralization of X mol% include two types, namely sodium acrylate (molecular weight: 94.04) and acrylic acid (molecular weight: 72.06), and therefore the molecular weight of the monomer units constituting sodium polyacrylate with a degree of neutralization of X mol% is regarded as the weighted average of the molecular weights of these two constituent units. Specifically, the molecular weight of the monomer units constituting sodium polyacrylate with a degree of neutralization of X mol% is a value calculated by {94.04 × X/100} + {72.06 × (100 - X)/100}. Therefore, the molecular weight of the monomer units constituting sodium polyacrylate with a degree of neutralization of 75 mol% is 88.5. Then, A (mol) is calculated by dividing the amount (g) of the water-absorbent resin particles subjected to cleavage by 88.5. Also, in the above equation, B is the total amount (mol) of monomer units constituting the precipitated solid polymer, and in the examples and comparative examples of the present application, the crosslinked structure of the water-absorbent resin particles manufactured by Sumitomo Seika Chemicals Co., Ltd. (Aqua Keep, sodium polyacrylate with a degree of neutralization of 75 mol%) is cleaved using a NaOH aqueous solution as described below, and the degree of neutralization is adjusted to 100 mol%, 75 mol%, or 50 mol%, and therefore B (mol) corresponds to the total amount (mol) of monomer units constituting the precipitated solid polymer, which is sodium polyacrylate. Therefore, B (mol) is calculated by dividing the total weight (g) of the precipitated solid polymer (sodium polyacrylate) by the molecular weight of the monomer units constituting sodium polyacrylate (in the case of a degree of neutralization of 100 mol%: 94.04, in the case of a degree of neutralization of 75 mol%: 88.55, in the case of a degree of neutralization of 50 mol%: 83.05). The total weight (g) of the precipitated solid polymer (sodium polyacrylate) is calculated by (c × d/100) × (1/{1 + (b₂/a₂)}), since the total solid content of the precipitated solid polymer (c × d/100) (g) contains the polymer (sodium polyacrylate) and impurities in a mass ratio of 1:(b₂/a₂).

### [Time Required]

In a case where the poor solvent was added dropwise to the polymer solution, the time for dropwise addition of the poor solvent was defined as the time required, and in a case where ultrafiltration was performed, the time for performing the ultrafiltration was defined as the time required.

### (Example 1)

A 4 L round-bottomed cylindrical separable flask (manufactured by DURAN) equipped with a reflux condenser, a dropping funnel, a nitrogen gas introduction pipe, and a stirring blade having four inclined paddle blades with a blade diameter of 100 mm in two stages as a stirrer was prepared, and 90 g of water-absorbent resin particles (manufactured by Sumitomo Seika Chemicals Co., Ltd., Aqua Keep HP600, sodium polyacrylate with a degree of neutralization of 75 mol%, unused product) were weighed therein, and 900 g of artificial urine (a mixture of 100 parts by mass of sodium chloride, 3 parts by mass of calcium chloride dihydrate, 6 parts by mass of magnesium chloride hexahydrate, 25 parts by mass of a 1% Triton aqueous solution, and 9866 parts by mass of ion-exchanged water), 810 g of ion-exchanged water, and 1200 g of a 1.5 mol/kg NaOH aqueous solution were added, and the mixture was stirred at 160 rpm while the internal temperature was maintained at 80°C, and allowed to react for 24 hours to cleave the crosslinked structure of the water-absorbent resin, thereby obtaining a polymer solution. The amount of the NaOH aqueous solution used was determined so as to be greater than an amount required for the degree of neutralization of the sodium polyacrylate with a degree of neutralization of 75 mol% contained in the polymer solution to exceed 100 mol%.

Next, 40 g of the polymer solution was placed in a 300 mL polybeaker, and stirred at 1000 rpm using a stirrer (manufactured by Shinto Scientific Co., Ltd., product name "BL1200", four stainless steel inclined paddle blades with a blade diameter of 50 mm). While stirring, 50.7 mL (40 g) of ethanol (manufactured by Hayashi Pure Chemical Ind., Ltd., special grade ethanol (99.5)) as a poor solvent was added dropwise at 10 mL/min (19.8 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in the form of small particles. The polymer dispersion liquid was filtered through a standard sieve with an opening of 75 µm, the supernatant liquid was discarded, and the precipitate remaining on the sieve was allowed to stand on the standard sieve with an opening of 75 µm for 30 minutes to drain, thereby obtaining a solid polymer.

### (Example 2)

A 4 L round-bottomed cylindrical separable flask (manufactured by DURAN) equipped with a reflux condenser, a dropping funnel, a nitrogen gas introduction pipe, and a stirring blade having four inclined paddle blades with a blade diameter of 100 mm in two stages as a stirrer was prepared, and 30 g of water-absorbent resin particles (manufactured by Sumitomo Seika Chemicals Co., Ltd., Aqua Keep HP600, degree of neutralization of 75%, unused product) were weighed therein, and 1200 g of artificial urine, 1370 g of ion-exchanged water, and 400 g of a 1.5 mol/kg NaOH aqueous solution were added, and the mixture was stirred at 160 rpm while the internal temperature was maintained at 80°C, and allowed to react for 24 hours to cleave the crosslinked structure of the water-absorbent resin, thereby obtaining a polymer solution. The amount of the NaOH aqueous solution used was determined so as to be greater than an amount required for the degree of neutralization of the sodium polyacrylate with a degree of neutralization of 75 mol% contained in the polymer solution to exceed 100 mol%.

Next, 40 g of the polymer solution was placed in a 300 mL polybeaker, and stirred at 1000 rpm using a stirrer (manufactured by Shinto Scientific Co., Ltd., product name "BL1200", four stainless steel inclined paddle blades with a blade diameter of 50 mm). While stirring, 50.7 mL (40 g) of ethanol (manufactured by Hayashi Pure Chemical Ind., Ltd., special grade ethanol (99.5)) as a poor solvent was added dropwise at 10 mL/min (19.8 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in the form of small particles. The supernatant liquid was discarded, and the precipitate was allowed to stand on a standard sieve with an opening of 75 µm for 30 minutes to drain, thereby obtaining a solid polymer.

### (Example 3)

A 4 L round-bottomed cylindrical separable flask (manufactured by DURAN) equipped with a reflux condenser, a dropping funnel, a nitrogen gas introduction pipe, and a stirring blade having four inclined paddle blades with a blade diameter of 100 mm in two stages as a stirrer was prepared, and 150 g of water-absorbent resin particles (manufactured by Sumitomo Seika Chemicals Co., Ltd., Aqua Keep HP600, sodium polyacrylate with a degree of neutralization of 75 mol%, unused product) were weighed therein, and 750 g of artificial urine, 100 g of ion-exchanged water, and 2000 g of a 1.5 mol/kg NaOH aqueous solution were added, and the mixture was stirred at 160 rpm while the internal temperature was maintained at 80°C, and allowed to react for 24 hours to cleave the crosslinked structure of the water-absorbent resin, thereby obtaining a polymer solution. The amount of the NaOH aqueous solution used was determined so as to be greater than an amount required for the degree of neutralization of the sodium polyacrylate with a degree of neutralization of 75 mol% contained in the polymer solution to exceed 100 mol%.

Next, 40 g of the polymer solution was placed in a 300 mL polybeaker, and stirred at 1000 rpm using a stirrer (manufactured by Shinto Scientific Co., Ltd., product name "BL1200", four stainless steel inclined paddle blades with a blade diameter of 50 mm). While stirring, 50.7 mL (40 g) of ethanol (manufactured by Hayashi Pure Chemical Ind., Ltd., special grade ethanol (99.5)) as a poor solvent was added dropwise at 10 mL/min (19.8 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in the form of small particles. The supernatant liquid was discarded, and the precipitate was allowed to stand on a standard sieve with an opening of 75 µm for 30 minutes to drain, thereby obtaining a solid polymer.

### (Example 4)

A solid polymer was precipitated and a polymer dispersion liquid was obtained in the same manner as in Example 1, except that the amount of ethanol added dropwise was changed to 12.7 mL (10 g). The shape of the precipitate was confirmed to be in the form of small particles. The supernatant liquid was discarded, and the precipitate was allowed to stand on a standard sieve with an opening of 75 µm for 30 minutes to drain, thereby obtaining a solid polymer.

### (Example 5)

A solid polymer was precipitated and a polymer dispersion liquid was obtained in the same manner as in Example 1, except that methanol (manufactured by FUJIFILM Wako Pure Chemical Corporation, reagent special grade) was used as a poor solvent instead of ethanol, and the amount of methanol added dropwise was changed to 50.6 mL (40 g). The shape of the precipitate was confirmed to be in the form of small particles. The supernatant liquid was discarded, and the precipitate was allowed to stand on a standard sieve with an opening of 75 µm for 30 minutes to drain, thereby obtaining a solid polymer.

### (Example 6)

A solid polymer was precipitated and a polymer dispersion liquid was obtained in the same manner as in Example 1, except that acetone (manufactured by FUJIFILM Wako Pure Chemical Corporation, reagent special grade) was used as a poor solvent instead of ethanol, and the amount of acetone added dropwise was changed to 21.6 mL (17.1 g). The shape of the precipitate was confirmed to be in the form of small particles. The supernatant liquid was discarded, and the precipitate was allowed to stand on a standard sieve with an opening of 75 µm for 30 minutes to drain, thereby obtaining a solid polymer.

### (Example 7)

A polymer solution was obtained in the same manner as in Example 1. 40 g of the polymer solution was placed in a 300 mL polybeaker, and 1.24 g of 95% sulfuric acid was added while stirring at 100 rpm using a stirrer (manufactured by Shinto Scientific Co., Ltd., product name "BL1200", four stainless steel inclined paddle blades with a blade diameter of 50 mm). Next, the rotation speed of the stirrer was changed to 100 rpm, and while stirring, 53.2 mL (42 g) of ethanol (manufactured by Hayashi Pure Chemical Ind., Ltd., special grade ethanol (99.5)) as a poor solvent was added dropwise at 10 mL/min (19.8 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in a lump shape. The supernatant liquid was discarded, and the precipitate was allowed to stand on a standard sieve with an opening of 75 µm for 30 minutes to drain, thereby obtaining a solid polymer. The amount of sulfuric acid used was determined so that the degree of neutralization of the sodium polyacrylate with a degree of neutralization of 100 mol% contained in the polymer solution became 75 mol%.

### (Example 8)

A polymer solution was obtained in the same manner as in Example 1. 40 g of the polymer solution was placed in a 300 mL polybeaker, and 1.41 g of 95% sulfuric acid was added while stirring at 100 rpm using a stirrer (manufactured by Shinto Scientific Co., Ltd., product name "BL1200", four stainless steel inclined paddle blades with a blade diameter of 50 mm). Next, the rotation speed of the stirrer was changed to 100 rpm, and while stirring, 54.0 mL (43 g) of ethanol (manufactured by Hayashi Pure Chemical Ind., Ltd., special grade ethanol (99.5)) as a poor solvent was added dropwise at 10 mL/min (19.8 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in a lump shape. The supernatant liquid was discarded, and the precipitate was allowed to stand on a standard sieve with an opening of 75 µm for 30 minutes to drain, thereby obtaining a solid polymer. The amount of sulfuric acid used was determined so that the degree of neutralization of the sodium polyacrylate with a degree of neutralization of 100 mol% contained in the polymer solution became 50 mol%.

### (Example 9)

A 1 L round-bottomed cylindrical separable flask (manufactured by Shibata Scientific Technology Ltd.) equipped with a reflux condenser, a dropping funnel, a nitrogen gas introduction pipe, and a stirring blade having four inclined paddle blades with a blade diameter of 100 mm in two stages as a stirrer was prepared, and 40 g of water-absorbent resin particles (manufactured by Sumitomo Seika Chemicals Co., Ltd., Aqua Keep HP600, sodium polyacrylate with a degree of neutralization of 75 mol%, unused product) were weighed therein, and 125 g of artificial urine, 68.25 g of ion-exchanged water, and 266.75 g of a 3 mol/kg NaOH aqueous solution were added, and the mixture was stirred at 50 rpm while the internal temperature was maintained at 80°C, and allowed to react for 24 hours to cleave the crosslinked structure of the water-absorbent resin, thereby obtaining a polymer solution. The amount of the NaOH aqueous solution used was determined so as to be greater than an amount required for the degree of neutralization of the sodium polyacrylate with a degree of neutralization of 75 mol% contained in the polymer solution to exceed 100 mol%.

Next, 40 g of the polymer solution was placed in a 300 mL polybeaker, and stirred at 1000 rpm using a stirrer (manufactured by Shinto Scientific Co., Ltd., product name "BL1200", four stainless steel inclined paddle blades with a blade diameter of 50 mm). While stirring, 21.7 mL (17.1 g) of ethanol (manufactured by Hayashi Pure Chemical Ind., Ltd., special grade ethanol (99.5)) as a poor solvent was added dropwise at 3.3 mL/min (6.6 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in the form of small particles. The supernatant liquid was discarded, and the precipitate was allowed to stand on a standard sieve with an opening of 75 µm for 30 minutes to drain, thereby obtaining a solid polymer.

### (Example 10)

A 1 L round-bottomed cylindrical separable flask (manufactured by Shibata Scientific Technology Ltd.) equipped with a reflux condenser, a dropping funnel, a nitrogen gas introduction pipe, and a stirring blade having four inclined paddle blades with a blade diameter of 100 mm in two stages as a stirrer was prepared, and 50 g of water-absorbent resin particles (manufactured by Sumitomo Seika Chemicals Co., Ltd., Aqua Keep HP600, sodium polyacrylate with a degree of neutralization of 75 mol%, unused product) were weighed therein, and 100 g of artificial urine, 16.75 g of ion-exchanged water, and 333.25 g of a 3 mol/kg NaOH aqueous solution were added, and the mixture was stirred at 50 rpm while the internal temperature was maintained at 80°C, and allowed to react for 24 hours to cleave the crosslinked structure of the water-absorbent resin, thereby obtaining a polymer solution. The amount of the NaOH aqueous solution used was determined so as to be greater than an amount required for the degree of neutralization of the sodium polyacrylate with a degree of neutralization of 75 mol% contained in the polymer solution to exceed 100 mol%.

Next, 40 g of the polymer solution was placed in a 300 mL polybeaker, and stirred at 1000 rpm using a stirrer (manufactured by Shinto Scientific Co., Ltd., product name "BL1200", four stainless steel inclined paddle blades with a blade diameter of 50 mm). While stirring, 21.7 mL (17.1 g) of ethanol (manufactured by Hayashi Pure Chemical Ind., Ltd., special grade ethanol (99.5)) as a poor solvent was added dropwise at 3.3 mL/min (6.6 mass%/min with respect to the polymer solution) using a pump (manufactured by Yamato Scientific Co., Ltd., Master flex, easy load 7518-16) to precipitate a solid polymer in the form of small particles, thereby obtaining a polymer dispersion liquid. After the dropwise addition of ethanol, stirring was stopped and the shape of the precipitate was visually confirmed to be in a lump shape. The supernatant liquid was discarded, and the precipitate was allowed to stand on a standard sieve with an opening of 75 µm for 30 minutes to drain, thereby obtaining a solid polymer.

### (Comparative Example 1)

A polymer solution was obtained in the same manner as in Example 1. The polymer solution was diluted 30-fold with pure water. The diluted polymer solution was placed in an ultrafiltration device, and the reservoir was filled with pure water. Filtration was started under the following conditions at 0.15 MPa and with stirring at 100 rpm. Sampling was performed periodically, and filtration was carried out until the impurity removal rate reached 75% or more. After 2880 minutes of filtration, a polymer solution containing a polymer having a weight-average molecular weight of 1,949,000 was obtained, and the impurity removal rate was 80.8% by mass and a polymer yield was 89% by mass.
· Ultrafiltration device: UHP-150K (Advantec)
· Reservoir: RP-1, RP-2 (Advantec)
· Ultrafiltration membrane (Synder, PX, 4000 MWCO, 0.0154)
· Filtration area: 0.0154 m²
· Molecular weight cut-off of ultrafiltration membrane: 400,000

**[Table 1]**

| | Polymer concentration in polymer solution [mass%] | Degree of neutralization of polymer [mol%] | Viscosity of polymer solution [mPa·s] | Concentration of poor solvent in polymer dispersion liquid [mass%] | Solid content fraction of precipitate [mass%] | Impurity removal rate [mass%] | Polymer yield [mol%] | Weight-average molecular weight [10,000] | Time required [min] |
|---|---|---|---|---|---|---|---|---|---|
| Example 1 | 3 | 100 | 10620 | 50 | 36 | 80 | 87 | 198.9 | 5.1 |
| Example 2 | 1 | 100 | 1737 | 50 | 33 | 88 | 92 | 194.4 | 5.1 |
| Example 3 | 5 | 100 | 29130 | 50 | 36 | 82 | 97 | 211.4 | 5.1 |
| Example 4 | 3 | 100 | 10620 | 20 | 24 | 86 | 93 | 195.8 | 1.3 |
| Example 5 | 3 | 100 | 10620 | 50 | 29 | 93 | 98 | 216.2 | 5.1 |
| Example 6 | 3 | 100 | 10620 | 30 | 25 | 85 | 93 | 234.2 | 2.1 |
| Example 7 | 3 | 75 | 1852 | 50 | 27 | 77 | 81 | 199.1 | 5.3 |
| Example 8 | 3 | 50 | 100 | 50 | 26 | 86 | 68 | 203.9 | 5.4 |
| Example 9 | 8 | 100 | 70940 | 30 | 33 | 87 | 93 | 222.7 | 6.3 |
| Example 10 | 10 | 100 | 114400 | 30 | 30 | 67 | 96 | 192.0 | 6.3 |

Examples 1 to 10 and Comparative Example 1 are experimental systems designed assuming that a solid polymer is obtained from water-absorbent resin particles used in diapers. Therefore, the polymer solution was prepared by cleaving the crosslinked structure of the water-absorbent resin particles using a NaOH aqueous solution in a state where the water-absorbent resin particles had absorbed artificial urine.

In Examples 1 to 10, a high-purity solid polymer could be obtained in a shorter time than in Comparative Example 1. In addition, in Examples 7 and 8, after the solid polymer precipitated as small particles, the small-particle solid polymers aggregated to form a lump shape. On the other hand, in Example 10, a lump-shaped solid polymer precipitated due to phase separation between the polymer solution and the poor solvent.

### Reference Signs List

1: water-absorbent resin particles, 10: absorbent body, 20a, 20b: core wrap sheets, 30: liquid permeable sheet, 40: liquid impermeable sheet, 50: water-absorbent sheet, 100: absorbent article.

## Claims

1. A production method for a solid polymer, the production method comprising:
a step of preparing a polymer solution comprising a polymer in which a crosslinked structure of a water-absorbent resin has been cleaved; and
a step of bringing a poor solvent for the polymer into contact with the polymer solution to precipitate the polymer, thereby obtaining a polymer dispersion liquid in which the solid polymer is dispersed.

2. The production method according to claim 1,
wherein the degree of neutralization of the polymer is 80 mol% or more.

3. The production method according to claim 1,
wherein the viscosity of the polymer solution at 25°C is 10 mPa·s to 200000 mPa·s.

4. The production method according to claim 1,
wherein the content of the poor solvent in the polymer dispersion liquid is 60% by mass or less.

5. The production method according to claim 1,
wherein the poor solvent comprises at least one selected from the group consisting of alcohols having 1 to 10 carbon atoms, phenols, and ketones.

6. A production method for water-absorbent resin particles, the production method comprising a step of crosslinking the solid polymer obtained by the production method according to any one of claims 1 to 5 to obtain a water-absorbent resin.
